# EUROPEAN PATENT APPLICATION

(11) **EP 2 365 082 A1**
(43) Date of publication of application: **14.09.2011**
(21) Application number: 10181849.0
(22) Date of filing: 27.06.2000
(51) Int. Cl.: C12N 15/40, C12N 7/04, C12N 15/869, A61K 39/12

(54) **BVDV virus-like particles**

(62) Divisional of application: 00113088.9
(71) Applicant: Pfizer Animal Health S.A., 1348 Ottignies, (BE); ID-Lelystad B.V., 8219 PH Lelystad (NL)
(72) Inventor: Schlapp, Tobias, 51061 Köln (DE); Rijsewijk, Franciscus Antonius Maria, 8244 AK Lelystad (NL)
(74) Representative: Becker, Philippe

(57) **Abstract**

The present invention relates to bovine viral diarrhea virus (BVDV) virus-like particles, a polycistronic RNA and DNA corresponding thereto encoding a polyprotein of BVDV structural proteins that are sufficient to form BVDV virus-like particles, a viral vector encoding factors and structural proteins for the assembly of BVDV virus-like particles, a vaccine comprising BVDV virus-like particles, a diagnostic kit and methods for preparing BVDV virus-like particles.

## Description

The present invention relates to bovine viral diarrhea virus (BVDV) virus-like particles, a polycistronic RNA and DNA corresponding thereto encoding a polyprotein of BVDV structural proteins that are sufficient to form BVDV virus-like particles, a viral vector encoding factors and structural proteins for the assembly of BVDV virus-like particles, a vaccine comprising BVDV virus-like particles, a diagnostic kit and methods for preparing BVDV virus-like particles.

Bovine viral diarrhea virus (BVDV) is the etiological agent of bovine viral diarrhea in cattle and has a world wide distribution and a prevalence that can be as high as 90%.

BVDV is a member of the genus pestivirus of the *Flaviviridae* family [Horzinek (1991), Pestiviruses - taxonomic perspectives, Arch. Virology Suppl. 3, 55-65]. BVDV has a positive-stranded RNA genome of approximately 12.5 kilo bases (kb), coding for one open reading frame which can be translated into one large polyprotein [Collet et al. (1988), Proteins encoded by bovine viral diarrhea virus: the genomic organisation of a pestivirus, Virology 165, 200-208]. A BVDV virion consists of a genomic RNA fitted into a nucleocapsid and this capsid is surrounded by an envelope containing glycoproteins.

The structural proteins, nucleocapsid protein (N) and three envelope glycoproteins (E^{rns}or gp48, E1 or gp25 and E2 or gp53) are found in this order close to the N terminal end of the polyprotein. All three glycoproteins are preceded by a signal peptide sequence and are liberated from the polyprotein in the lumen of the endoplasmatic reticulum (ER) by host signal peptidases.

For a related *Flavivirus,* hepatitis C virus, expression of a genome segment which encodes only the structural proteins is sufficient to form virus-like particles [Baumert et al. (1998), Hepatitis C virus structural proteins assemble into virus-like particles in insect cells, J. Virol. 72, 3827-3836].

Bovine herpesvirus 1 (BHV-1) is the etiological agent of infectious bovine rhinotracheitis (IBR) and of infectious pustular vulvovaginitis (IPV) and infectious balanopostitis (IBP). BHV-1 is found in cattle all over the world with high prevalences.

BHV-1 is a member of the alphaherpesviruses, has a double stranded DNA genome of about 136 kilo base pairs and codes for about 70 genes of which about 30 genes are non essential for the replication of the virus. A spontaneous mutant with a deletion of the non essential glycoprotein E (gE) gene is used in safe and efficacious marker vaccines [Kaashoek et al. (1994), A conventionally attenuated glycoprotein E-negative strain of bovine herpesvirus type 1 is an efficacious and safe vaccine, Vaccine 12, 439-444].

Vaccines to protect against BVDV induced diseases are based on either inactivated BVDV strains, on subunit vaccines based on isolated BVDV glycoproteins, on attenuated BVDV strains [reviewed by Van Oirschot et al. (1999), Vaccination of cattle against bovine viral diarrhoea, Vet. Microbiol. 64, 169-183], on DNA vaccines [Reddy et al. (1999), Comp Immunol Microbiol Infect Dis. 22, 231-246; Harpin et al. (1999), Vaccination of cattle with a DNA plasmid encoding the bovine viral diarrhoea virus major glycoprotein E2, J. Gen. Virol. 80, 3137-3144], or on vector vaccines. Vaccines based on attenuated BVDV strains may cause immunosuppression and in utero infections [Roth & Kaeberle (1983), Suppression of neutrophil and lymphocyte function induced by a vaccinal strain of bovine viral diarrhea virus with or without administration of ACTH, Am. J. Vet. Res. 44, 2366-2372; Liess et al. (1984), Studies on transplacental transmissibility of a bovine virus diarrhea (BVD) vaccine virus in cattle, Zentrbl. Veterinarmed. Reihe B 31, 669-681] and may be less efficacious in the presence of maternal antibodies. Vaccines based on inactivated BVDV or subunit vaccines are less efficacious because the antigens are not produced intracellularly and therefore less efficiently presented to the T cell compartment of the immune system. E.g. two out of three baculovirus BVDV E2 subunit vaccines did not protect against a foetal BVDV infection in a sheep model [Bruschke et al. (1997), A subunit vaccine based on glycoprotein E2 of bovine virus diarrhea virus induces fetal protection in sheep against homologous challenge, Vaccine 15, 1940-1945].

DNA vaccines and vector vaccines do not have this disadvantage but the presently used DNA and vector vaccines are only expressing part of the structural proteins and fail to form the more immunogenic virus-like particles. E.g. WO 95/12682 describes the expression of only BVDV E2 in the TK locus of a BHV-1 strain [cf. also Kweon et al. (1999), Bovine herpesvirus expressing envelope protein (E2) of bovine viral diarrhea virus as a vaccine candidate, J. Vet. Med. Sci. 61, 395-401].

BVDV shows antigenic variation and can be divided in several antigenic groups (BVDV IA and IB, and BVDV II). This antigenic variation is mainly based on sequence variations in E2 [van Rijn et al. (1997), Subdivision of the pestivirus genus based on envelope glycoprotein E2, Virology 237, 337-348].

Vaccines based on E2 of only one BVDV type are more restricted in their cross protection [Bolin and Ridpath (1996), Glycoprotein E2 of bovine viral diarrhea virus expressed in insect cells provides calves limited protection from systemic infection and disease, Arch. Virol. 141, 1463-1477] than vaccines that express also the less variable N, E^{ms} and E1 proteins [Elahi et al. (1999), Induction of humoral and cellular immune responses against the nucleocapsid of bovine viral diarrhea virus by an adenovirus vector with an inducible promoter, Virology 261, 1-7].

The present invention provides BVDV virus-like particles.

The term "virus-like particles" as used herein refers to particles composed of most or all structural proteins of a virus that have a large part of the structural characteristics in common with their infectious wild type counterparts. However, upon interaction with the host cell, virus-like particles do not produce progeny viruses, because the proper nucleic acid sequences are not present in these particles. In case of BVDV virus-like particles, the nucleocapsids may be empty or may contain irrelevant RNA, but their overall structure is like wild type nucleocapsids and the surrounding envelope contains the same transmembrane glycoproteins E1 and E2 and the same membrane associated protein E^{rns}, as are found in wild type BVDV.

In particular, the virus-like particles according to the present invention comprise the BVDV structural proteins N, E^{rns}, E1 and E2. The invention contemplates BVDV virus-like particles which can be derived from various naturally occurring BVDV strains such as BVDV type I A strains (represented by the NADL strain), BVDV type I B strains (represented by the Osloss strain) and BVDV type II strains (represented by the 890 strain) including the cytopathic strain 87-2552 [Reddy et al. (1995), Antigenic differences between a field isolate and vaccine strains of bovine viral diarrhea virus, J. Clin. Microbiol. 33, 2159-2161] and BVDV type I strain PT810, used as an example in this application. The present invention also contemplates BVDV virus-like particles comprising BVDV structural proteins which are not identical to naturally occurring proteins but contain amino acid substitutions, deletions and/or insertions provided that the mutant structural proteins retain the capability of being assembled into virus-like particles.

The BVDV structural proteins are derived from a polyprotein which is processed after translation. To prepare the BVDV virus-like particles according to the present invention it is favourable to use host cells which contain the information for synthesising the structural proteins from a DNA template. A cDNA prepared from naturally occurring RNA which codes for the polyprotein of BVDV structural proteins would not work because mRNA transcribed from such a DNA template contains too many splice sites that would be recognised by spliceosomes in the cell nucleus. RNA transcribed from such cDNA would be destroyed before it can be translated.

The present invention, therefore, provides polycistronic RNA molecules comprising a ribonucleotide sequence which codes for the BVDV structural proteins N, E^{rns}, E1 and E2, which RNA is scarcely or not at all being spliced in the cell nucleus within its polyprotein encoding part.

A preferred embodiment of the RNA molecules according to the present invention is represented by RNA comprising a ribonucleotide sequence which codes for a polyprotein having the amino acid sequence according to SEQ ID NO: 2 and which does not contain strong potential splice sites within its polyprotein encoding part, i.e. no potential splice acceptor sites with a score above -22 and no potential splice donor sites with a score above -13.1.

A most preferred embodiment of the RNA molecules according to the present invention is represented by a RNA molecule which comprises a ribonucleotide sequence corresponding to the polynucleotide sequence from Nucleotide No. 17 to Nucleotide No. 2710 according to SEQ ID NO: 1.

The inventive RNA molecules can be obtained from corresponding DNA fragments which are also provided by the present invention.

The DNA fragments according to the present invention code for the BVDV structural proteins N, E^{rns}, E1 and E2, but do not contain strong potential splice sites. Splice sites are recognition sequences in eukaryotic mRNAs that are either exon-intron junctions (splice donor sequences) or intron-exon junctions (splice acceptor sequences) and are used by spliceosomes in the nucleus to remove introns from pre-mRNAs to fuse coding regions that are located on exons together to form a complete open reading frame. BVDV cDNA contains 'accidental' splicing signals that are never used because BVDV RNA normally stays in the cytoplasm. By expressing BVDV cDNA via viral vectors such as BHV1, the RNA will be made in the nucleus and be processed by the spliceosome [Shiu et al. (1997), The presence of RNA splicing signals in the cDNA construct of the E2 gene of classical swine fever virus affected its expression, J. Virol. Methods 69, 223-230].

To remove most of the (potential) splicing signals from the BVDV cDNA encoding N, E^{rns}, E1 and E2, the nucleic acid and protein sequence analysis software system of PC/Gene version 2.32 Jan. 1989 can be used. In this software program the option "splice junctions" which is based on the method of Staden [(1984), Computer methods to locate signals in nucleic acid sequences, Nucl. Acids. Res. 12, 505-519] is appropriate to be used in the present invention.

A preferred embodiment of the DNA fragments according to the present invention is represented by a DNA comprising the polynucleotide sequence from Nucleotide No. 17 to Nucleotide No. 2710 according to SEQ ID NO: 1.

The present invention further provides DNA constructs suitable to produce BVDV virus-like particles. In order to allow the BVDV structural proteins to be expressed in host cells the DNA is to be operably linked to cis-regulatory sequences. Such regulatory sequences are capable of binding RNA polymerases in a cell and of initiating transcription of a downstream (3' direction) coding sequence. For purposes of defining the present invention, the cis-regulatory sequences are followed at the 3' terminus by a Kozak consensus sequence and the translation start codon (ATG) of a coding sequence and extend upstream (5' direction) to include the minimum number of bases or elements necessary to initiate transcription at levels detectable above background. Within the cis-regulatory sequences will be found a transcription initiation site as well as protein binding motifs responsible for the binding of RNA polymerase II complexes. The choice of such regulatory sequences is obvious to those skilled in the art. Suitable cis-regulatory sequences can be derived from eukaryotic genes, preferably from mammals, or from genes from viruses that infect mammals. Example are the simian virus 40 (SV40) promoter and the cis-regulatory sequences located on the long terminal repeats of retroviruses such as the murine leukemia virus (MuLV) long terminal repeat (MLV-LTR).

In a preferred embodiment of the DNA constructs according to the present invention the DNA encoding the polyprotein of BVDV structural proteins is operably linked to the human cytomegalovirus immediate early 1 promoter.

The DNA constructs according to the present invention preferably further comprise terminator sequences at the 3' terminus of the coding region. mRNA synthesis by RNA polymerase II in eukaryotic cells has a clear starting point, generally at a short distance downstream of the 'TATA-box', but no clear stop signal. The poly(A) signal found upstream of the 3' end of the transcribed region plays an essential role in the cleavage and polyadenylation of the 3' end of mRNAs [Keller (1995), No end yet to messenger RNA 3' processing, Cell 81, 829-832] and is also implicated in termination of transcription, but sequences downstream of this poly(A) signal are thought to play a role in the termination of the mRNA transcription as well. These sequences are implicated in retarding or pausing the transcription complex [Vandenbergh (1991), An apparent pause site in the transcription unit of the rabbit alpha-globin gene, J. Mol. Biol. 220, 255-270]. As efficient termination and polyadenylation confers stability to mRNAs, termination signals -like the one found downstream of the bovine growth hormone gene- are often included in eukaryotic expression vectors.

In a preferred embodiment of the DNA constructs according to the present invention the DNA encoding the polyprotein of BVDV structural proteins is followed by the bovine growth hormone terminator sequence.

From a practical standpoint the use of viral vectors is favourable for the following reasons:
● Viral vectors can express the proteins encoded on inserted gene(s) at high levels;
● the recombinant viruses can be produced to high titres in suitable host cells at lower costs than subunit vaccines;
● upon administration to the host the recombinant viruses express the proteins encoded on inserted gene(s), producing high amounts of these proteins in the host;
● the proteins encoded by the inserted gene(s) are expressed intracellularly and can therefore efficiently be presented by MHC class I molecules to stimulate the cytotoxic T cells of the host;
● all the structural proteins of the BVDV genome can be expressed and form virus-like particles, comparable to modified live virus vaccine, but without the risks normally encountered with such vaccines, like e.g. reversion to wild-type;
● live vaccines are generally more immunogenic than killed vaccines.

The present invention provides a viral vector encoding factors and BVDV structural proteins necessary for the assembly of BVDV virus-like particles.

In a preferred embodiment the viral vector is represented by bovine herpesvirus 1 (BHV-1).

In a further preferred embodiment a BHV-1 vector which contains a deletion within the sequence coding for glycoprotein E (gE) or which completely lacks the genome region coding for gE is used for preparing BVDV virus-like particles. An example for such a BHV-1 vector is Difivac-1 deposited under Accession No. I-1213 with the Institut Pasteur, France. The use of such vector allows the production of a BHV-1/BVDV vaccine with double marker properties. Hosts, in particular cattle, immunised with the BHV-1/BVDV vaccine described herein form antibodies against BHV-1 and BVDV, but not against BHV-1 gE or the BHV-1gI/gE complex and not against the BVDV non structural proteins, in particular NS3 or p80. This allows discrimination of hosts, in particular cattle, infected with wild-type BHV-1 and/or wild-type BVDV from hosts only vaccinated with this BVH-1BVDV vaccine, by measuring the presence of anti-BHV-1gE or anti-BHV-1gI/gE antibodies and/or anti-BVDV antibodies in their body fluids, in particular nasal fluid samples and serum samples.

A most preferred embodiment of the viral vector according to the present invention is represented by A9-SV-1F9 (synth. BVDV capsid; E^{rns}, E1 and E2 in gE locus of Difivac-1) deposited under CNCM accession No. I-2488 with the Collection Nationale de Cultures de Microorganismes, Institut Pasteur (25, Rue du Docteur Roux, F-75724 Paris, France) on June 8, 2000.

The present invention further provides host cells containing the above-mentioned viral vectors. The viral vectors can be introduced into the host cells by infection or transfection. Examples of suitable host cells are embryonic bovine trachea (EBTr) cells or Madin-Darby bovine kidney (MDBK) cells.

The present invention also provides a vaccine for immunising a host against BVDV induced diseases which comprises BVDV virus-like particles and a pharmaceutically acceptable carrier or diluent. Examples of pharmaceutically acceptable carriers or diluents useful in the present invention include stabilizers such as carbohydrates (e.g. sorbitol, mannitol, starch, sucrose, glucose, dextran), proteins such as albumin or casein, protein containing agents such as bovine serum or skimmed milk and buffers (e.g. phosphate buffer). Optionally, one or more compounds having adjuvant activity may be added to the vaccine. Suitable adjuvants are for example aluminium hydroxide, phosphate or oxide and oil emulsions such as saponins.

The useful effective amount to be administered will vary depending on the age, weight, mode of administration and type of pathogen against which vaccination is sought. A suitable dosage can be for example about 10³-10⁷ pfu/animal.

The vaccine according to the present invention can be given inter alia intranasally, intradermally, subcutaneously or intramuscularly.

A preferred vaccine according to the present invention comprises a BHV-1BVDV recombinant viral vector which will lead to the intracellular production of BVDV virus-like particles in the vaccinated host and will induce beneficial immune responses to protect against BVDV induced disease. Moreover, such a vaccine will also protect against BHV-1 induced disease and, if BHV-1 lacking gE is used as a vector, hosts vaccinated with such a vaccine can be discriminated from hosts infected either with BHV-1 or with BVDV.

The present invention further provides a diagnostic kit for detecting the presence and/or absence of anti-BVDV antibodies and, in particular, for detecting the presence and/or absence of anti-BVDV and anti-BHV-1 antibodies in a biological sample such as a biological fluid sample, in particular a nasal fluid sample or a serum sample. These diagnostic tests are used to discriminate between hosts infected with wild type virus and hosts only vaccinated with the BHV-1BVDV vaccine described herein.

The anti-BHV-1 and/or anti-BVDV antibodies detected in these diagnostic tests are reacting with viral antigens that are not present in the vaccine, in particular BHV-1gE and the BHV-1 gI/gE complex and the BVDV non structural proteins, in particular NS3 or p80.

The present invention further provides a method for preparing BVDV virus-like particles comprising
(a) inserting a DNA construct as set forth above into a viral vector encoding factors for the assembly of BVDV virus-like particles,
(b) infecting suitable host cells capable of expressing the polyprotein encoded by the DNA, and
(c) culturing the host cells under appropriate conditions.

The present invention also provides a method for preparing BVDV virus-like particles comprising
(a) infecting suitable host cells with a viral vector as set forth above, and
(b) culturing the host cells under appropriate conditions.

The present invention further provides a method for preparing BHV1/BVDV recombinant viruses that encode all structural proteins for the formation of BVDV virus-like particles comprising
(a) inserting a DNA construct as set forth above into the genome of BHV-1,
(b) infecting suitable host cells capable of expressing the polyprotein encoded by the vector, and
(c) culturing the host cells under appropriate conditions.

Suitable host cells can be infected with the recombinant viruses by adding the supernatant of an infectious culture to a monolayer of these cells, e.g. EBTr cells or MDBK cells. For example on a monolayer in a 150 cm² tissue culture flask, after removal of the medium, 1 to 3 ml of virus containing (titres ranging from 10³ to 10⁷ pfu/ml) culture medium can be added. After two hours incubation fresh tissue culture medium can be added up to 30 ml. Two to three days after infection full cytopathogenic effect (cpe) will be visible and up to 10⁸ pfu/ml can be found in the medium.

### Examples

### Construction of the coding region for BVDV structural proteins

To determine the amino acid sequences of the structural proteins of a recent BVDV isolate, the 5' half of the nucleotide sequence of BVDV type I strain PT810, which has recently been isolated in Europe, was established using standard methods.

The coding regions for the nucleocapsid protein N and the glycoproteins, E^{rns}, E1 and E2 were identified by comparing the deduced amino acid sequences with published BVDV sequences. These coding regions contained sequences (splicing signals) which, when expressed by BHV-1 in the nucleus, could be recognized and processed by spliceosomes. Therefore, the sequence was adapted in such a way that most of the (potential) splicing signals were removed while the coding potential remained unaffected. The adapted sequence has been made synthetically and codes for the same amino acids as the originally BVDV sequence with the exception of the first amino acid of the N protein.

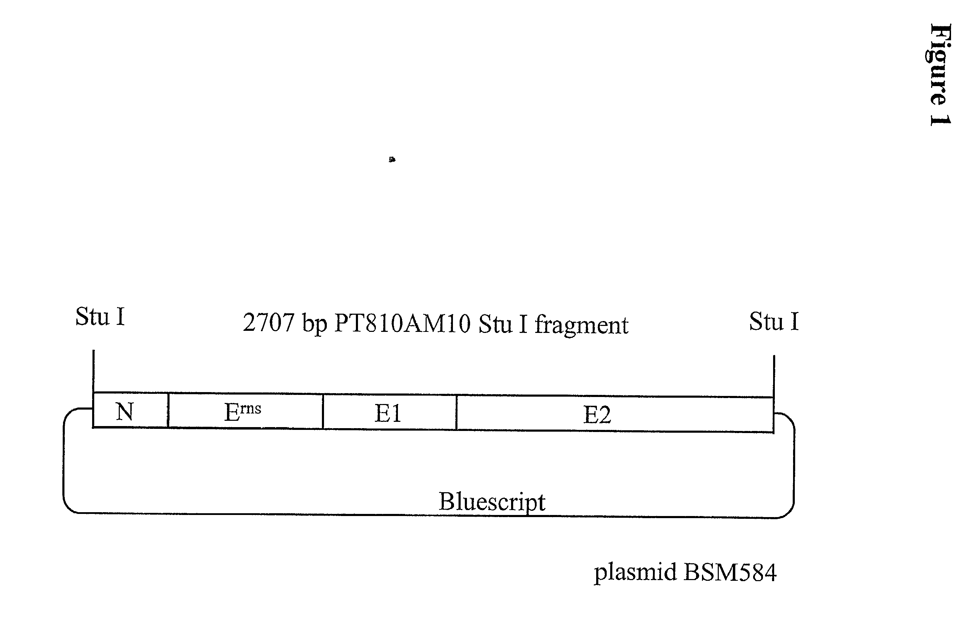

In BVDV strain PT810 the first amino acid of the N protein is a serine but to allow efficient translation of the synthetic coding region, this amino acid was replaced by a methionine and preceded by a Kozak consensus sequence. To end the protein encoded region, a stop codon was inserted behind the putative carboxy- terminal end of the E2 protein, right after its transmembrane region. To allow easy cloning, the sequence recognised by the Stu I restriction enzyme, was added to both sides of the synthetic sequence bringing the total length to 2715 nucleotides. See SEQ ID NO: 1. A double stranded DNA fragment has been synthesised and cloned into prokaryotic plasmid Bluescript and propagated in *Escherichia coli* bacteria. See Figure 1.

### Construction of the BHV-1BVDV recombination/expression cassette for the gE locus of Difivac-1

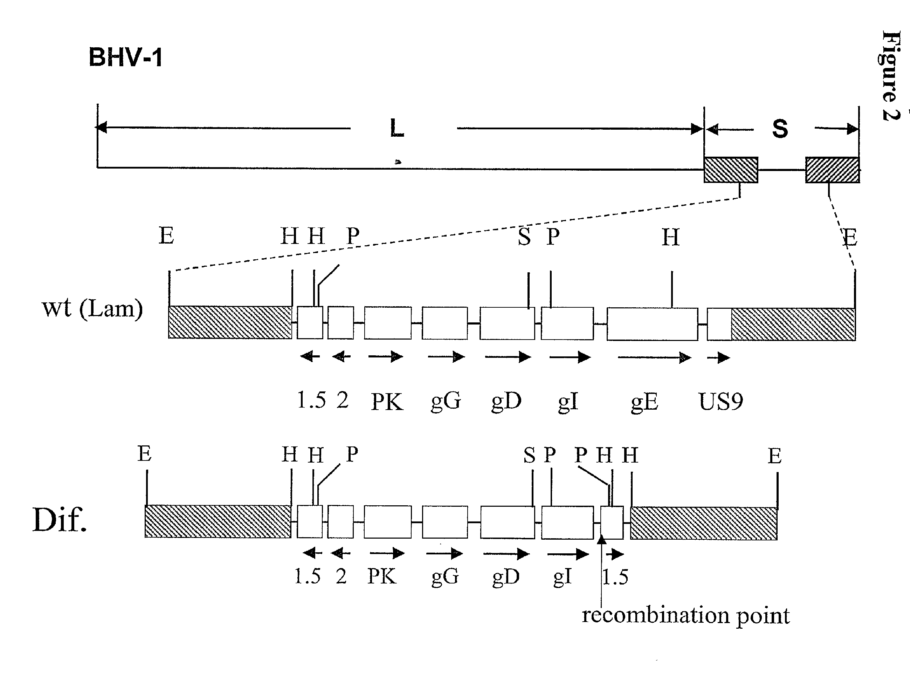

Analysis of the recombination found in the U_{S} region of the gE deleted Za strain (which has been renamed Difivac-1 in WO 92/21751) showed the deletion of the glycoprotein E gene and the neighbouring US9 gene and a concomitant duplication inversion of part of the US 1.5 gene. This analysis also showed the position of the recombination point [Rijsewijk et al. (1999), Spontaneous BHV-1 recombinants in which the gI/gE/US9 region is replaced by a duplication/inversion of the US1.5/US2 region, Arch. Virol. 144, 1527-1537]. See Figure 2.

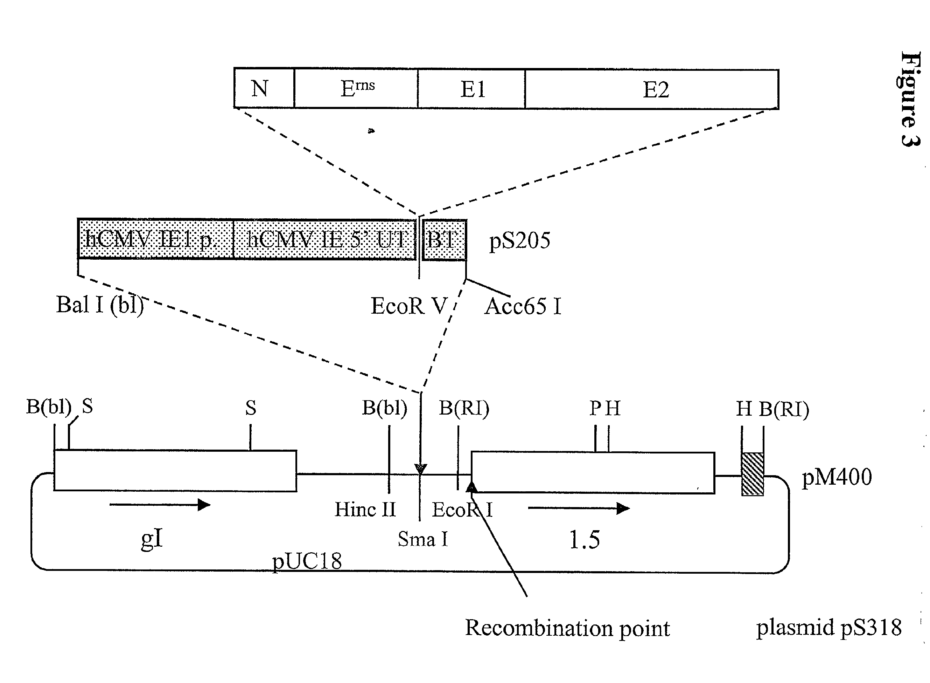

To insert heterologous genes in the genome of the Za strain at or close to the position of the gE deletion, a recombination cassette has been constructed. To this end, a 0.8 kilo base pair Bsa I fragment encoding part of the gI gene and ending 40 bp upstream of the Za recombination point and a 0.7 kilo base pair Bsa I fragment starting 40 bp downstream of the Za recombination point and encoding part of the US1.5 gene, have been cloned in pUC18 in their original orientation. This construct has been named pM400 and has between the recombination fragments a unique Sma I site for the insertion of the expression cassette. See Figure 3.

In the Sma I site of pM400 a 1.9 kilo base pair Bal I ― Acc65 I fragment from plasmid pVR1012 has been cloned with its cis-regulatory sequences in the same orientation as the flanking recombination fragments. These cis-regulatory sequences are the human cytomegalovirus immediate early 1 promoter (hCMVIEp.) and its 5' untranslated leader (5'UT), and the bovine growth hormone terminator sequence (BT). The resulting construct has been named pS205 and has a unique EcoR V site between the promoter region and the terminator region.

In the EcoR V site of pS205 the 2707 base pair Stu I fragment, encoding the BVDV nucleocapsid protein N and the BVDV glycoproteins, E^{rns}, E1 and E2, has been cloned in the same orientation as the promoter/terminator and the flanking recombination sequences. This BHV-1BVDV recombination/expression cassette has been named pS318. See Figure 3.

### Construction and isolation of BHV-1 recombinant virus expression the coding region for BVDV structural proteins

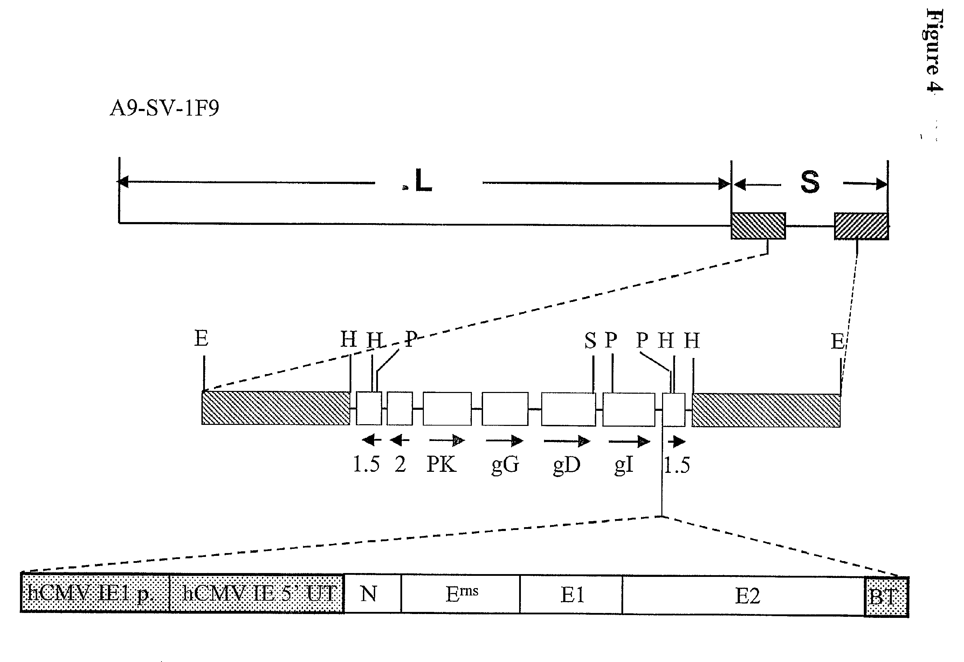

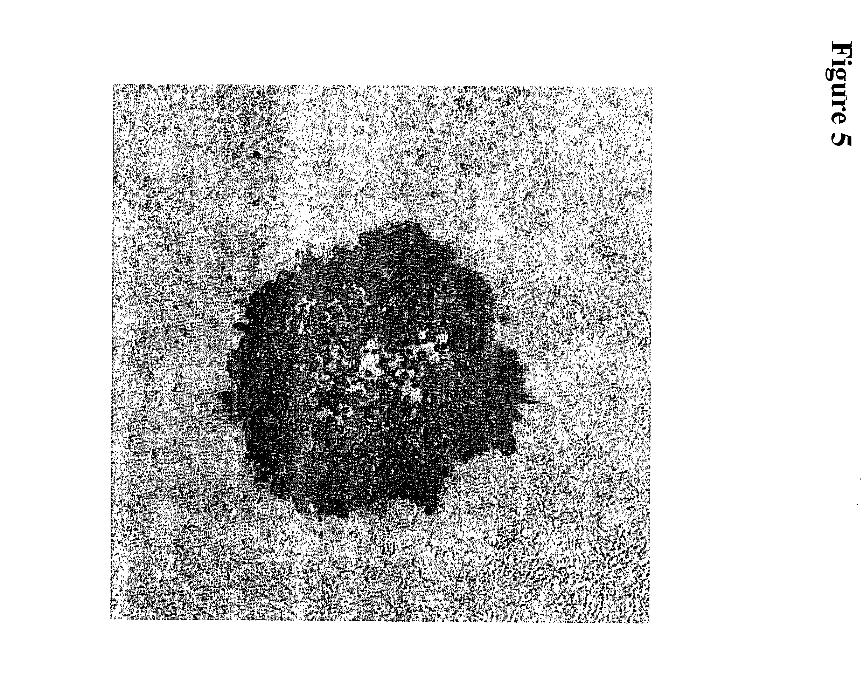

To construct the BHV-1/BVDV recombinant (as described in Figure 4) that expresses the BVDV nucleocapsid protein N and the BVDV glycoproteins, E^{rns}, E1 and E2, linearized pS318 plasmid and genomic DNA of the Za strain were cotransfected into bovine cells according to standard methods and 48 hours after cotransfection cells were freeze/thawed to liberate putative recombinant viruses. To isolate these recombinant viruses, the cell debris was pelleted and the supernatant was used to infect bovine cells on a 96 wells plate and to identify BVDV positive wells using an immunostaining procedure with anti-BVDV E2 MAb 166. The virus in the medium of BVDV E2 positive wells was diluted to infect bovine cells on another 96 wells microplate and virus from BVDV E2 positive single plaques was three times plaque purified to obtain a purified recombinant. One of the recombinants obtained this way, was named A9-SV-1F9 and passaged 8 times on MDBK cells to test the stability of the expression on a panel of six different bovine cell lines. See Figure 5. In all bovine cells types tested, even after 8 passages, virtually all plaques were found BVDV E2 positive. In MDBK cells the percentage of BVDV E2 positive plaques was actually determined and found to be after 8 passages still more than 90%.

### Legends to Sequence Listing and Figure

### SEQ ID NOs: 1 and 2

The 2715 nucleotides long sequence of the synthetic DNA fragment PT810AM10 which codes for proteins N, E^{rns}, E1 and E2 of BVDV strain PT810. To both sides of the DNA fragment the recognition sequence of restriction enzyme Stu I is added and upstream of the start codon (ATG) a Kozak consensus sequences is inserted. The PT810AM10 sequence has been translated using the universal genetic code and the encoded amino acid sequence has been indicated in the three letter code below the nucleotide sequence. All amino acids encoded by the open reading frame are identical to the ones found in BVDV strain PT810, with exception of the first amino acid of the open reading frame. This first amino acid is a serine residue in PT810 and was changed into a methionine residue in PT810AM10. The stop codon at the end of the protein coding region overlaps with the Stu I site at the end of the DNA fragment.

### SEQ ID NOs: 3 and 4

Segment of SEQ ID NO: 1 coding for protein N.

### SEQ ID NOs: 5 and 6

Segment of SEQ ID NO: 1 coding for protein E^{rns}.

### SEQ ID NOS: 7 and 8

Segment of SEQ ID NO: 1 coding for protein E1.

### SEQ ID NOs: 9 and 10

Segment of SEQ ID NO: 1 coding for protein E2.

### Figure 1

Structure of plasmid BSM584. The 2715 nucleotides long DNA fragment PT810AM10 has been cloned in prokaryotic plasmid Bluescript. By digesting plasmid BSM584 with restriction enzyme Stu I a 2707 nucleotides long fragment was liberated that was inserted into the BHV-1 recombination/expression cassette pS205. See Figure 3.

### Figure 2

Top: Diagram of the structure of the BHV-1 genome and the recombination found in the Za or Difivac-1 strain that has been used as a vector. Wild type BHV-1 is approximately 136 kilo base pares long and has a Long (L) and a short (S) segment. The short fragment has a unique domain bordered by an inverted repeat indicated by hatched boxes.

Middle: In unique short (Uₛ) domain 8 open reading frames have been recognised: US 1.5, US2, PK, gG, gD, gI gE and US9.

Bottom: In the natural gE deletion mutant Za or Difivac-1 (Dif.) the gE and US9 genes have been deleted and part of the US 1.5 gene has been duplicated instead. The recombination point has been indicated by an arrow. This Difivac-1 mutant has been described in WO 92/21751 and in Arch. Virol. (1999) 144, 1527-1537 by Rijsewijk et al.

### Figure 3

Diagram of the recombination/expression cassette plasmid pS318. The 0.8 kilo base pairs Bsa I (B) fragment from the upstream side of the gE locus and the 0.7 kilo base pairs Bsa I fragment from the downstream side of the gE locus, both isolated from the Difivac-1 strain, have been cloned in respectively the Hinc II site and the EcoR I site of prokaryotic plasmid pUC18. To insert the 0.8 kbp Bsa I fragment into the Hinc II site the Bsa I fragment has been made blunt ended and to insert the 0.7 kbp Bsa I fragment into the EcoR I site EcoR I linkers have been added to this fragment using standard methods. The resulting plasmid has been named pM400. In the Sma I site of pM400 a fragment taken from plasmid pVR1012 containing the IE1 promoter/enhancer of the human cytomegalovirus (hCMVIEp.) and the 5' untranslated region of the same promoter (5'UT) and the bovine growth hormone terminator (BT) sequence, has been inserted. The resulting plasmid has been named pS205. Into the unique EcoR V site of pS205 the 2707 bp Stu I fragment from plasmid BSM584 has been inserted. The resulting clone has been named plasmid pS318 and this plasmid has been used in cotransfection experiments to recombine the BVDV genes into the gE locus of Za (Difivac-1). See Figure 4.

### Figure 4

Diagram of the BHV-1BVDV recombinant A9-SV-1F9 that encodes all structural proteins of BVDV strain PT810. On the top the structure of the BHV-1 genome has been indicated. In the middle the EcoR I fragment that includes the unique short region with the position of all genes and on the bottom the BVDV expression cassette with the 2707 bp long fragment encoding the structural proteins of BVDV stain PT810 have been indicated. The BVDV open reading frame has been preceded by the human cytomegalovirus IE1 promoter/enhancer region (hCMVIE1p.) and the 5' untranslated (5'UT) leader of this promoter and the BVDV open reading frame has been followed by the bovine growth hormone terminator sequence (BT). The cassette has been inserted into the Difivac-1 genome 40 bp upstream of the recombination site found in this genome at the gE locus in the same orientation as the surrounding genes.

### Figure 5

IPMA of a monolayer of MDBK cells infected with BHV-1BVDV recombinant A9-SV-1F9 and stained with anti- BVDV E2 MAb 166 four days after infection. The infected cells form a round plaque that is stained by the antibody while the surrounding cells are not stained.

## Claims

1. BVDV virus-like particles.

2. BVDV virus-like particles according to claim 1 comprising the BVDV structural proteins N, E^{rns}, E1 and E2.

3. Polycistronic RNA molecule comprising a ribonucleotide sequence encoding a polyprotein consisting of the BVDV structural proteins N, E^{rns}, E1 and E2, said RNA molecule being not spliced in the cell nucleus within its polyprotein encoding part.

4. RNA molecule according to claim 3 encoding a polyprotein having the amino acid sequence according to SEQ ID NO: 2 provided that said RNA molecule does not contain strong potential splice sites.

5. RNA molecule according to claim 4 comprising a ribonucleotide sequence corresponding to the polynucleotide sequence from Nucleotide No. 17 to Nucleotide No. 2710 according to SEQ ID NO: 1

6. DNA fragment corresponding to the RNA molecule according to any of claims 3 to 5.

7. DNA fragment according to claim 6 comprising the polynucleotide sequence from Nucleotide No. 17 to Nucleotide No. 2710 according to SEQ ID NO: 1.

8. DNA construct comprising the DNA according to claim 6 or 7 operably linked to cis-regulatory sequences capable of controlling the expression of the polyprotein encoded by said DNA.

9. DNA construct according to claim 8 further comprising a terminator sequence.

10. DNA construct according to claim 8 or 9 wherein the cis-regulatory sequences are derived from the human cytomegalovirus immediate early 1 promoter and 5' untranslated leader.

11. DNA construct according to claim 9 wherein the terminator sequence is derived from the bovine growth hormone terminator sequence.

12. Viral vector encoding factors for the assembly of BVDV virus-like particles, said viral vector comprising the DNA construct according to any of claims 8 to 11.

13. Viral vector according to claim 12 which is BHV-1 or a BHV-1 deletion mutant.

14. Viral vector according to claim 13 which is Difivac-1 deposited under Accession No. I-1213.

15. Viral vector according to any of claims 12 to 14 wherein said BHV-1 vector carries said DNA construct within the sequence coding for glycoprotein gE or at the position within a mutant BHV-1 vector where the sequence coding for glycoprotein gE is deleted.

16. Viral vector according to claim 15 which is A9-SV-1F9 deposited under the CNCM accession No. I-2488.

17. Host cell containing the vector according to any of claims 12 to 16.

18. Vaccine comprising BVDV virus-like particles according to claim 1 or 2 and a pharmaceutically acceptable carrier or diluent.

19. Vaccine according to claim 18 further comprising BHV-1.

20. Vaccine according to claim 19 wherein said BHV-1 lacks glycoprotein gE.

21. Vaccine according to claim 20 wherein said BHV-1 is Difivac-1 deposited under CNCM accession No. I-1213.

22. Vaccine comprising recombinant viruses encoded by and including the vector according to any of claims 12 to 16 and a pharmaceutically acceptable carrier or diluent.

23. Diagnostic kit containing BVDV virus-like particles according to claim 1 or 2.

24. Diagnostic kit according to claim 23 further containing BVDV NS3 and/or BVDV p80 protein or immunogenic fragments thereof.

25. Diagnostic kit according to claim 23 or 24 further containing BHV-1 gE protein and/or BHV-1 gI/gE protein complex or immunogenic fragments thereof.

26. Method for preparing BVDV virus-like particles comprising
(a) inserting the DNA construct according to any of claims 8 to 11 into a viral vector encoding factors for the assembly of BVDV virus-like particles,
(b) infecting suitable host cells capable of expressing the polyprotein encoded by said DNA, and
(c) culturing said host cells under appropriate conditions.

27. Method for preparing recombinant viruses encoded by and including the vector according to any of claims 12 to 16 comprising
(a) infecting suitable host cells with a viral vector according to 12 to 16,
(b) culturing said host cells under appropriate conditions, and optionally
(c) isolating the recombinant viruses.

28. Method for preparing a vaccine according to claim 22 comprising admixing the recombinant viruses with a pharmaceutically acceptable carrier.


SEQUENCE LISTING

<110> Bayer AG
      ID-Lelystad B.V.

<120> BVDV virus-like particles

<130> Le A 34 440

<140>
<141>

<160> 10

<170> PatentIn Ver. 2.1

<210> 1
<211> 2715
<212> DNA
<213> Bovine viral diarrhea virus

<220>
<221> CDS
<222> (17)..(2707)

<220>
<221> 3'UTR
<222> (8)..(16)
<223> Kozak consensus sequence

<400> 1
aaggcctgcc gccacc atg gac acg aaa gaa gaa ggg gca aca aaa aaa caa 52
                  Met Asp Thr Lys Glu Glu Gly Ala Thr Lys Lys Gln
                    1               5                  10

cag aaa ccg gac cgc gtt gaa aaa ggg cgc atg aaa ata acg cct aaa   100
Gln Lys Pro Asp Arg Val Glu Lys Gly Arg Met Lys Ile Thr Pro Lys
         15                  20                  25

gaa act gaa aaa gat tcc cgg acc aaa cca cct gat gct acg atc gtc   148
Glu Thr Glu Lys Asp Ser Arg Thr Lys Pro Pro Asp Ala Thr Ile Val
     30                  35                  40

gtc gac ggc gtc aaa tac caa gtc aaa aaa aaa ggc aaa gtc aaa tcc   196
Val Asp Gly Val Lys Tyr Gln Val Lys Lys Lys Gly Lys Val Lys Ser
 45                  50                  55                  60

aaa aac acc caa gat ggg ctc tac cac aat aaa aat aaa cca caa gaa   244
Lys Asn Thr Gln Asp Gly Leu Tyr His Asn Lys Asn Lys Pro Gln Glu
                 65                  70                  75

tca cgc aaa aaa ctg gaa aaa gct cta ttg gct tgg gca ata ttg gct   292
Ser Arg Lys Lys Leu Glu Lys Ala Leu Leu Ala Trp Ala Ile Leu Ala
             80                  85                  90

gtt gta tta ttt caa gtc aca atg ggg gaa aac ata aca caa tgg aac   340
Val Val Leu Phe Gln Val Thr Met Gly Glu Asn Ile Thr Gln Trp Asn
         95                 100                 105

ttg caa gac aat gga acc gaa ggc gtc caa cgg gct atg ttt gaa cgc   388
Leu Gln Asp Asn Gly Thr Glu Gly Val Gln Arg Ala Met Phe Glu Arg
    110                 115                 120

ggc gtc aat cgg agc tta cat gga atc tgg ccc gaa aaa atc tgc acc 436
Gly Val Asn Arg Ser Leu His Gly Ile Trp Pro Glu Lys Ile Cys Thr
125                 130                 135                 140

ggc gtc cca tct cat ttg gcc acc gat atg gaa ttg aaa cga att cat 484
Gly Val Pro Ser His Leu Ala Thr Asp Met Glu Leu Lys Arg Ile His
                145                 150                 155

gga atg atg gac gca tct gaa aaa acc aac tat aca tgc tgc cgg ctt 532
Gly Met Met Asp Ala Ser Glu Lys Thr Asn Tyr Thr Cys Cys Arg Leu
            160                 165                 170

caa cga cat gaa tgg aat aaa cat ggc tgg tgc aat tgg tac aat atc 580
Gln Arg His Glu Trp Asn Lys His Gly Trp Cys Asn Trp Tyr Asn Ile
        175                 180                 185

gaa cct tgg att ctg ctt atg aat cgg acc caa gct aac ctc act gaa 628
Glu Pro Trp Ile Leu Leu Met Asn Arg Thr Gln Ala Asn Leu Thr Glu
    190                 195                 200

ggc caa cca caa cgc gaa tgc gcc gtc acc tgc cgc tat gac cgg aat 676
Gly Gln Pro Gln Arg Glu Cys Ala Val Thr Cys Arg Tyr Asp Arg Asn
205                 210                 215                 220

tcc gac ttg aat gtc gtg aca caa gcc cgg gac tct ccg aca cca ctt 724
Ser Asp Leu Asn Val Val Thr Gln Ala Arg Asp Ser Pro Thr Pro Leu
                225                 230                 235

acg gga tgc aaa aaa ggg aaa aac ttc tct ttt tcg ggc atc gtc atc 772
Thr Gly Cys Lys Lys Gly Lys Asn Phe Ser Phe Ser Gly Ile Val Ile
            240                 245                 250

caa ggc cct tgc aat ttt gaa att gct gca tct gac gtc ctc ttc aaa 820
Gln Gly Pro Cys Asn Phe Glu Ile Ala Ala Ser Asp Val Leu Phe Lys
        255                 260                 265

gaa cat gac tgc aca tcc ata ttt caa gat act gct cat tac ctc gtt 868
Glu His Asp Cys Thr Ser Ile Phe Gln Asp Thr Ala His Tyr Leu Val
    270                 275                 280

gat ggg atg act aac tct ttg gag tct gct cga caa gga act gca aaa 916
Asp Gly Met Thr Asn Ser Leu Glu Ser Ala Arg Gln Gly Thr Ala Lys
285                 290                 295                 300

cta aca act tgg ctg ggg cga caa ctt ggg ata ttg ggg aaa aaa ctg 964
Leu Thr Thr Trp Leu Gly Arg Gln Leu Gly Ile Leu Gly Lys Lys Leu
                305                 310                 315

gaa aac aaa tcc aaa aca tgg ttc ggg gct tat gca tct tcc cct tac 1012
Glu Asn Lys Ser Lys Thr Trp Phe Gly Ala Tyr Ala Ser Ser Pro Tyr
            320                 325                 330

tgc gat gtt gaa cga aaa ctt ggc tac atc tgg ttt aca aaa aat tgc 1060
Cys Asp Val Glu Arg Lys Leu Gly Tyr Ile Trp Phe Thr Lys Asn Cys
        335                 340                 345

acc cct gcc tgc ctc ccc aaa aac aca aaa att gtt gga cct ggg aaa 1108
Thr Pro Ala Cys Leu Pro Lys Asn Thr Lys Ile Val Gly Pro Gly Lys
    350                 355                 360

ttt gac acc aat gcc gaa gat gga aaa ata tta cat gaa atg ggg ggc 1156
Phe Asp Thr Asn Ala Glu Asp Gly Lys Ile Leu His Glu Met Gly Gly
365                 370                 375                 380

cac ctt tcg gaa gtt cta tta ctt tca ctt gtt gtt cta tcc gat ttc 1204
His Leu Ser Glu Val Leu Leu Leu Ser Leu Val Val Leu Ser Asp Phe
                385                 390                 395

gca ccc gaa act gcc tct gcg atg tat ctt gtc cta cat ttt tcc atc 1252
Ala Pro Glu Thr Ala Ser Ala Met Tyr Leu Val Leu His Phe Ser Ile
            400                 405                 410

cca caa cga cac acc gat gtt ctg gac tgc gat aaa tct caa cta aat 1300
Pro Gln Arg His Thr Asp Val Leu Asp Cys Asp Lys Ser Gln Leu Asn
        415                 420                 425

cta acc atg ggc gtc aca acc gcc gat gtt ata ccc gga tcc gtc tgg 1348
Leu Thr Met Gly Val Thr Thr Ala Asp Val Ile Pro Gly Ser Val Trp
    430                 435                 440

aat atg ggc aaa tat gtt tgc ata cga ccc gac tgg tgg cct tat gaa 1396
Asn Met Gly Lys Tyr Val Cys Ile Arg Pro Asp Trp Trp Pro Tyr Glu
445                 450                 455                 460

acg gct gct gtt ctg gct ttg gaa gaa gtt ggg caa gtt aca cgg atc 1444
Thr Ala Ala Val Leu Ala Leu Glu Glu Val Gly Gln Val Thr Arg Ile
                465                 470                 475

gtc ttg cgg gca ctc cgc gac ttg aca cgc atc tgg aac gct gcc aca 1492
Val Leu Arg Ala Leu Arg Asp Leu Thr Arg Ile Trp Asn Ala Ala Thr
            480                 485                 490

acc act gca ttt ctt gtc tgc ctt gtt aaa gtt gtc cgc gga caa gtc 1540
Thr Thr Ala Phe Leu Val Cys Leu Val Lys Val Val Arg Gly Gln Val
        495                 500                 505

tta caa ggc gtc ata tgg tta ctg cta ata acg ggc gtc caa gga cgc 1588
Leu Gln Gly Val Ile Trp Leu Leu Leu Ile Thr Gly Val Gln Gly Arg
    510                 515                 520

ctc gat tgc aaa cct gac ttc tca tat gcc att gcc aaa aat gaa aaa 1636
Leu Asp Cys Lys Pro Asp Phe Ser Tyr Ala Ile Ala Lys Asn Glu Lys
525                 530                 535                 540

att gga cca ctg ggg gct gaa gga ctt act acc act tgg tat gaa tac 1684
Ile Gly Pro Leu Gly Ala Glu Gly Leu Thr Thr Thr Trp Tyr Glu Tyr
                545                 550                 555

tct gat ggg atg caa ctt tcc gac act atg gtt gaa gct cga tgc aaa 1732
Ser Asp Gly Met Gln Leu Ser Asp Thr Met Val Glu Ala Arg Cys Lys
            560                 565                 570

gat ggg gaa ttt aca ttc atc caa aaa tgc aaa acg gaa acc cga tat 1780
Asp Gly Glu Phe Thr Phe Ile Gln Lys Cys Lys Thr Glu Thr Arg Tyr
        575                 580                 585

ctg gcc acc ttg cac aca cgg gcc tta ccg aca tct gtc gtt ttt gaa 1828
Leu Ala Thr Leu His Thr Arg Ala Leu Pro Thr Ser Val Val Phe Glu
    590                 595                 600

aaa ctt ttt gat gga aat aaa ttg gcg gac atc gtt gaa atg gat gac 1876
Lys Leu Phe Asp Gly Asn Lys Leu Ala Asp Ile Val Glu Met Asp Asp
605                 610                 615                 620

aac ttc gaa ttt gcg atc tgc ccc tgc gat gca aaa ccc gtc gtc cgc 1924
Asn Phe Glu Phe Ala Ile Cys Pro Cys Asp Ala Lys Pro Val Val Arg
                625                 630                 635

ggg aaa ttt aac aca aca cta cta aat ggg ccc gcc ttc caa atg gtc 1972
Gly Lys Phe Asn Thr Thr Leu Leu Asn Gly Pro Ala Phe Gln Met Val
            640                 645                 650

tgc ccc att gga tgg act gga tct gtc tcc tgc acc cta gcc aat aaa 2020
Cys Pro Ile Gly Trp Thr Gly Ser Val Ser Cys Thr Leu Ala Asn Lys
        655                 660                 665

gac acc ctc gat acg gcc gtc gtc cgg aca tat aaa cgc gtt tcc cca 2068
Asp Thr Leu Asp Thr Ala Val Val Arg Thr Tyr Lys Arg Val Ser Pro
    670                 675                 680

ttc cct aat cgg caa gga tgc gtt act caa aaa ctt ctc ggg gaa gat 2116
Phe Pro Asn Arg Gln Gly Cys Val Thr Gln Lys Leu Leu Gly Glu Asp
685                 690                 695                 700

ctt tat gat tgc atc ttg ggc gga aac tgg act tgc atc gaa ggg gaa 2164
Leu Tyr Asp Cys Ile Leu Gly Gly Asn Trp Thr Cys Ile Glu Gly Glu
                705                 710                 715

caa cta cga tac act ggg ggc acc att gaa tcc tgc aag tgg tgc ggc 2212
Gln Leu Arg Tyr Thr Gly Gly Thr Ile Glu Ser Cys Lys Trp Cys Gly
            720                 725                 730

tac aaa ttc ttg aaa tcg gaa ggg cta cca cac tat cca att ggc aaa 2260
Tyr Lys Phe Leu Lys Ser Glu Gly Leu Pro His Tyr Pro Ile Gly Lys
        735                 740                 745

tgc cgc tta caa aat gaa act ggc tac cgg ctt gtc gac gac acc tct 2308
Cys Arg Leu Gln Asn Glu Thr Gly Tyr Arg Leu Val Asp Asp Thr Ser
    750                 755                 760

tgc aat gtc ggc ggc gtc gca att gtc cca cat gga ctt gtc aaa tgc 2356
Cys Asn Val Gly Gly Val Ala Ile Val Pro His Gly Leu Val Lys Cys
765                 770                 775                 780

aaa att ggg gat acc gtc gtc caa gtc gtc gca atg gac acg aaa ctt 2404
Lys Ile Gly Asp Thr Val Val Gln Val Val Ala Met Asp Thr Lys Leu
                785                 790                 795

gga cct atg cct tgc aaa cca cat gaa ata ata tca tcg gaa gga ccc 2452
Gly Pro Met Pro Cys Lys Pro His Glu Ile Ile Ser Ser Glu Gly Pro
            800                 805                 810

gtt gaa aaa acg gca tgc aca ttc aac tat aca cgg acc tta acg aac 2500
Val Glu Lys Thr Ala Cys Thr Phe Asn Tyr Thr Arg Thr Leu Thr Asn
        815                 820                 825

aaa tat ttt gaa ccc cgg gac aat tac ttc caa caa tac atg cta aaa 2548
Lys Tyr Phe Glu Pro Arg Asp Asn Tyr Phe Gln Gln Tyr Met Leu Lys
    830                 835                 840

ggg gac tac caa tat tgg ttt gat ctg gaa gtc tct gac cac cat cgg 2596
Gly Asp Tyr Gln Tyr Trp Phe Asp Leu Glu Val Ser Asp His His Arg
845                 850                 855                 860

gat tac ttt acg gaa ttc cta ctt gtc att gtt gtc gcc ctc ttg ggc 2644
Asp Tyr Phe Thr Glu Phe Leu Leu Val Ile Val Val Ala Leu Leu Gly
                865                 870                 875

gga cgc tat gtc ctt tgg cta ctt gtc aca tac atg gtc ctc tcc gaa 2692
Gly Arg Tyr Val Leu Trp Leu Leu Val Thr Tyr Met Val Leu Ser Glu
            880                 885                 890

caa aat gcc tcg gct taggcctt 2715
Gln Asn Ala Ser Ala
        895

<210> 2
<211> 897
<212> PRT
<213> Bovine viral diarrhea virus

<400> 2
Met Asp Thr Lys Glu Glu Gly Ala Thr Lys Lys Gln Gln Lys Pro Asp
1               5                   10                  15

Arg Val Glu Lys Gly Arg Met Lys Ile Thr Pro Lys Glu Thr Glu Lys
            20                  25                  30

Asp Ser Arg Thr Lys Pro Pro Asp Ala Thr Ile Val Val Asp Gly Val
        35                  40                  45

Lys Tyr Gln Val Lys Lys Lys Gly Lys Val Lys Ser Lys Asn Thr Gln
    50                  55                  60

Asp Gly Leu Tyr His Asn Lys Asn Lys Pro Gln Glu Ser Arg Lys Lys
65                  70                  75                  80

Leu Glu Lys Ala Leu Leu Ala Trp Ala Ile Leu Ala Val Val Leu Phe
                85                  90                  95

Gln Val Thr Met Gly Glu Asn Ile Thr Gln Trp Asn Leu Gln Asp Asn
            100                 105                 110

Gly Thr Glu Gly Val Gln Arg Ala Met Phe Glu Arg Gly Val Asn Arg
        115                 120                 125

Ser Leu His Gly Ile Trp Pro Glu Lys Ile Cys Thr Gly Val Pro Ser
    130                 135                 140

His Leu Ala Thr Asp Met Glu Leu Lys Arg Ile His Gly Met Met Asp
145                 150                 155                 160

Ala Ser Glu Lys Thr Asn Tyr Thr Cys Cys Arg Leu Gln Arg His Glu
                165                 170                 175

Trp Asn Lys His Gly Trp Cys Asn Trp Tyr Asn Ile Glu Pro Trp Ile
            180                 185                 190

Leu Leu Met Asn Arg Thr Gln Ala Asn Leu Thr Glu Gly Gln Pro Gln
        195                 200                 205

Arg Glu Cys Ala Val Thr Cys Arg Tyr Asp Arg Asn Ser Asp Leu Asn
    210                 215                 220

Val Val Thr Gln Ala Arg Asp Ser Pro Thr Pro Leu Thr Gly Cys Lys
225                 230                 235                 240

Lys Gly Lys Asn Phe Ser Phe Ser Gly Ile Val Ile Gln Gly Pro Cys
                245                 250                 255

Asn Phe Glu Ile Ala Ala Ser Asp Val Leu Phe Lys Glu His Asp Cys
                260                 265                 270

Thr Ser Ile Phe Gln Asp Thr Ala His Tyr Leu Val Asp Gly Met Thr
            275                 280                 285

Asn Ser Leu Glu Ser Ala Arg Gln Gly Thr Ala Lys Leu Thr Thr Trp
        290                 295                 300

Leu Gly Arg Gln Leu Gly Ile Leu Gly Lys Lys Leu Glu Asn Lys Ser
305                 310                 315                 320

Lys Thr Trp Phe Gly Ala Tyr Ala Ser Ser Pro Tyr Cys Asp Val Glu
                325                 330                 335

Arg Lys Leu Gly Tyr Ile Trp Phe Thr Lys Asn Cys Thr Pro Ala Cys
                340                 345                 350

Leu Pro Lys Asn Thr Lys Ile Val Gly Pro Gly Lys Phe Asp Thr Asn
            355                 360                 365

Ala Glu Asp Gly Lys Ile Leu His Glu Met Gly Gly His Leu Ser Glu
        370                 375                 380

Val Leu Leu Leu Ser Leu Val Val Leu Ser Asp Phe Ala Pro Glu Thr
385                 390                 395                 400

Ala Ser Ala Met Tyr Leu Val Leu His Phe Ser Ile Pro Gln Arg His
                405                 410                 415

Thr Asp Val Leu Asp Cys Asp Lys Ser Gln Leu Asn Leu Thr Met Gly
                420                 425                 430

Val Thr Thr Ala Asp Val Ile Pro Gly Ser Val Trp Asn Met Gly Lys
            435                 440                 445

Tyr Val Cys Ile Arg Pro Asp Trp Trp Pro Tyr Glu Thr Ala Ala Val
        450                 455                 460

Leu Ala Leu Glu Glu Val Gly Gln Val Thr Arg Ile Val Leu Arg Ala
465                 470                 475                 480

Leu Arg Asp Leu Thr Arg Ile Trp Asn Ala Ala Thr Thr Thr Ala Phe
                485                 490                 495

Leu Val Cys Leu Val Lys Val Val Arg Gly Gln Val Leu Gln Gly Val
                500                 505                 510

Ile Trp Leu Leu Leu Ile Thr Gly Val Gln Gly Arg Leu Asp Cys Lys
            515                 520                 525

Pro Asp Phe Ser Tyr Ala Ile Ala Lys Asn Glu Lys Ile Gly Pro Leu
        530                 535                 540

Gly Ala Glu Gly Leu Thr Thr Thr Trp Tyr Glu Tyr Ser Asp Gly Met
545                 550                 555                 560

Gln Leu Ser Asp Thr Met Val Glu Ala Arg Cys Lys Asp Gly Glu Phe
                565                 570                 575

Thr Phe Ile Gln Lys Cys Lys Thr Glu Thr Arg Tyr Leu Ala Thr Leu
                580                 585                 590

His Thr Arg Ala Leu Pro Thr Ser Val Val Phe Glu Lys Leu Phe Asp
        595                 600                 605

Gly Asn Lys Leu Ala Asp Ile Val Glu Met Asp Asp Asn Phe Glu Phe
    610                 615                 620

Ala Ile Cys Pro Cys Asp Ala Lys Pro Val Val Arg Gly Lys Phe Asn
625                 630                 635                 640

Thr Thr Leu Leu Asn Gly Pro Ala Phe Gln Met Val Cys Pro Ile Gly
                645                 650                 655

Trp Thr Gly Ser Val Ser Cys Thr Leu Ala Asn Lys Asp Thr Leu Asp
            660                 665                 670

Thr Ala Val Val Arg Thr Tyr Lys Arg Val Ser Pro Phe Pro Asn Arg
        675                 680                 685

Gln Gly Cys Val Thr Gln Lys Leu Leu Gly Glu Asp Leu Tyr Asp Cys
    690                 695                 700

Ile Leu Gly Gly Asn Trp Thr Cys Ile Glu Gly Glu Gln Leu Arg Tyr
705                 710                 715                 720

Thr Gly Gly Thr Ile Glu Ser Cys Lys Trp Cys Gly Tyr Lys Phe Leu
                725                 730                 735

Lys Ser Glu Gly Leu Pro His Tyr Pro Ile Gly Lys Cys Arg Leu Gln
            740                 745                 750

Asn Glu Thr Gly Tyr Arg Leu Val Asp Asp Thr Ser Cys Asn Val Gly
        755                 760                 765

Gly Val Ala Ile Val Pro His Gly Leu Val Lys Cys Lys Ile Gly Asp
    770                 775                 780

Thr Val Val Gln Val Val Ala Met Asp Thr Lys Leu Gly Pro Met Pro
785                 790                 795                 800

Cys Lys Pro His Glu Ile Ile Ser Ser Glu Gly Pro Val Glu Lys Thr
                805                 810                 815

Ala Cys Thr Phe Asn Tyr Thr Arg Thr Leu Thr Asn Lys Tyr Phe Glu
            820                 825                 830

Pro Arg Asp Asn Tyr Phe Gln Gln Tyr Met Leu Lys Gly Asp Tyr Gln
        835                 840                 845

Tyr Trp Phe Asp Leu Glu Val Ser Asp His His Arg Asp Tyr Phe Thr
    850                 855                 860

Glu Phe Leu Leu Val Ile Val Val Ala Leu Leu Gly Gly Arg Tyr Val
865                 870                 875                 880

Leu Trp Leu Leu Val Thr Tyr Met Val Leu Ser Glu Gln Asn Ala Ser
                885                 890                 895

Ala

<210> 3
<211> 303
<212> DNA
<213> Bovine viral diarrhea virus

<220>
<221> CDS
<222> (1)..(303)
<223> Protein N

<400> 3
atg gac acg aaa gaa gaa ggg gca aca aaa aaa caa cag aaa ccg gac     48
Met Asp Thr Lys Glu Glu Gly Ala Thr Lys Lys Gln Gln Lys Pro Asp
1               5                   10                  15

cgc gtt gaa aaa ggg cgc atg aaa ata acg cct aaa gaa act gaa aaa     96
Arg Val Glu Lys Gly Arg Met Lys Ile Thr Pro Lys Glu Thr Glu Lys
            20                  25                  30

gat tcc cgg acc aaa cca cct gat gct acg atc gtc gtc gac ggc gtc     144
Asp Ser Arg Thr Lys Pro Pro Asp Ala Thr Ile Val Val Asp Gly Val
        35                  40                  45

aaa tac caa gtc aaa aaa aaa ggc aaa gtc aaa tcc aaa aac acc caa     192
Lys Tyr Gln Val Lys Lys Lys Gly Lys Val Lys Ser Lys Asn Thr Gln
    50                  55                  60

gat ggg ctc tac cac aat aaa aat aaa cca caa gaa tca cgc aaa aaa     240
Asp Gly Leu Tyr His Asn Lys Asn Lys Pro Gln Glu Ser Arg Lys Lys
65                  70                  75                  80

ctg gaa aaa gct cta ttg gct tgg gca ata ttg gct gtt gta tta ttt     288
Leu Glu Lys Ala Leu Leu Ala Trp Ala Ile Leu Ala Val Val Leu Phe
                85                  90                  95

caa gtc aca atg ggg                                                 303
Gln Val Thr Met Gly
            100


<210> 4
<211> 101
<212> PRT
<213> Bovine viral diarrhea virus

<400> 4
Met Asp Thr Lys Glu Glu Gly Ala Thr Lys Lys Gln Gln Lys Pro Asp
1               5                   10                  15

Arg Val Glu Lys Gly Arg Met Lys Ile Thr Pro Lys Glu Thr Glu Lys
            20                  25                  30

Asp Ser Arg Thr Lys Pro Pro Asp Ala Thr Ile Val Val Asp Gly Val
        35                  40                  45

Lys Tyr Gln Val Lys Lys Lys Gly Lys Val Lys Ser Lys Asn Thr Gln
    50                  55                  60

Asp Gly Leu Tyr His Asn Lys Asn Lys Pro Gln Glu Ser Arg Lys Lys
65                  70                  75                  80

Leu Glu Lys Ala Leu Leu Ala Trp Ala Ile Leu Ala Val Val Leu Phe
                85                  90                  95

Gln Val Thr Met Gly
            100

<210> 5
<211> 681
<212> DNA
<213> Bovine viral diarrhea virus

<220>
<221> CDS
<222> (1)..(681)
<223> Protein ERNS

<400> 5
gaa aac ata aca caa tgg aac ttg caa gac aat gga acc gaa ggc gtc     48
Glu Asn Ile Thr Gln Trp Asn Leu Gln Asp Asn Gly Thr Glu Gly Val
  1               5                  10                  15

caa cgg gct atg ttt gaa cgc ggc gtc aat cgg agc tta cat gga atc     96
Gln Arg Ala Met Phe Glu Arg Gly Val Asn Arg Ser Leu His Gly Ile
             20                  25                  30

tgg ccc gaa aaa atc tgc acc ggc gtc cca tct cat ttg gcc acc gat    144
Trp Pro Glu Lys Ile Cys Thr Gly Val Pro Ser His Leu Ala Thr Asp
         35                  40                  45

atg gaa ttg aaa cga att cat gga atg atg gac gca tct gaa aaa acc    192
Met Glu Leu Lys Arg Ile His Gly Met Met Asp Ala Ser Glu Lys Thr
     50                  55                  60

aac tat aca tgc tgc cgg ctt caa cga cat gaa tgg aat aaa cat ggc    240
Asn Tyr Thr Cys Cys Arg Leu Gln Arg His Glu Trp Asn Lys His Gly
 65                  70                  75                  80

tgg tgc aat tgg tac aat atc gaa cct tgg att ctg ctt atg aat cgg    288
Trp Cys Asn Trp Tyr Asn Ile Glu Pro Trp Ile Leu Leu Met Asn Arg
                 85                  90                  95

acc caa gct aac ctc act gaa ggc caa cca caa cgc gaa tgc gcc gtc    336
Thr Gln Ala Asn Leu Thr Glu Gly Gln Pro Gln Arg Glu Cys Ala Val
            100                 105                 110

acc tgc cgc tat gac cgg aat tcc gac ttg aat gtc gtg aca caa gcc    384
Thr Cys Arg Tyr Asp Arg Asn Ser Asp Leu Asn Val Val Thr Gln Ala
        115                 120                 125

cgg gac tct ccg aca cca ctt acg gga tgc aaa aaa ggg aaa aac ttc    432
Arg Asp Ser Pro Thr Pro Leu Thr Gly Cys Lys Lys Gly Lys Asn Phe
    130                 135                 140

tct ttt tcg ggc atc gtc atc caa ggc cct tgc aat ttt gaa att gct    480
Ser Phe Ser Gly Ile Val Ile Gln Gly Pro Cys Asn Phe Glu Ile Ala
145                 150                 155                 160

gca tct gac gtc ctc ttc aaa gaa cat gac tgc aca tcc ata ttt caa    528
Ala Ser Asp Val Leu Phe Lys Glu His Asp Cys Thr Ser Ile Phe Gln
                165                 170                 175

gat act gct cat tac ctc gtt gat ggg atg act aac tct ttg gag tct    576
Asp Thr Ala His Tyr Leu Val Asp Gly Met Thr Asn Ser Leu Glu Ser

180                 185                 190

gct cga caa gga act gca aaa cta aca act tgg ctg ggg cga caa ctt     624
Ala Arg Gln Gly Thr Ala Lys Leu Thr Thr Trp Leu Gly Arg Gln Leu
        195                 200                 205

ggg ata ttg ggg aaa aaa ctg gaa aac aaa tcc aaa aca tgg ttc ggg     672
Gly Ile Leu Gly Lys Lys Leu Glu Asn Lys Ser Lys Thr Trp Phe Gly
    210                 215                 220

gct tat gca                                                         681
Ala Tyr Ala
225


<210> 6
<211> 227
<212> PRT
<213> Bovine viral diarrhea virus

<400> 6
Glu Asn Ile Thr Gln Trp Asn Leu Gln Asp Asn Gly Thr Glu Gly Val
1               5                   10                  15

Gln Arg Ala Met Phe Glu Arg Gly Val Asn Arg Ser Leu His Gly Ile
            20                  25                  30

Trp Pro Glu Lys Ile Cys Thr Gly Val Pro Ser His Leu Ala Thr Asp
        35                  40                  45

Met Glu Leu Lys Arg Ile His Gly Met Met Asp Ala Ser Glu Lys Thr
    50                  55                  60

Asn Tyr Thr Cys Cys Arg Leu Gln Arg His Glu Trp Asn Lys His Gly
65                  70                  75                  80

Trp Cys Asn Trp Tyr Asn Ile Glu Pro Trp Ile Leu Leu Met Asn Arg
                85                  90                  95

Thr Gln Ala Asn Leu Thr Glu Gly Gln Pro Gln Arg Glu Cys Ala Val
            100                 105                 110

Thr Cys Arg Tyr Asp Arg Asn Ser Asp Leu Asn Val Val Thr Gln Ala
        115                 120                 125

Arg Asp Ser Pro Thr Pro Leu Thr Gly Cys Lys Lys Gly Lys Asn Phe
    130                 135                 140

Ser Phe Ser Gly Ile Val Ile Gln Gly Pro Cys Asn Phe Glu Ile Ala
145                 150                 155                 160

Ala Ser Asp Val Leu Phe Lys Glu His Asp Cys Thr Ser Ile Phe Gln
                165                 170                 175

Asp Thr Ala His Tyr Leu Val Asp Gly Met Thr Asn Ser Leu Glu Ser
            180                 185                 190

Ala Arg Gln Gly Thr Ala Lys Leu Thr Thr Trp Leu Gly Arg Gln Leu
        195                 200                 205

Gly Ile Leu Gly Lys Lys Leu Glu Asn Lys Ser Lys Thr Trp Phe Gly
    210                 215                 220

Ala Tyr Ala
225

<210> 7
<211> 585
<212> DNA
<213> Bovine viral diarrhea virus

<220>
<221> CDS
<222> (1)..(585)
<223> Protein E1

<400> 7
tct tcc cct tac tgc gat gtt gaa cga aaa ctt ggc tac atc tgg ttt 48
Ser Ser Pro Tyr Cys Asp Val Glu Arg Lys Leu Gly Tyr Ile Trp Phe
1               5                   10                  15

aca aaa aat tgc acc cct gcc tgc ctc ccc aaa aac aca aaa att gtt 96
Thr Lys Asn Cys Thr Pro Ala Cys Leu Pro Lys Asn Thr Lys Ile Val
            20                  25                  30

gga cct ggg aaa ttt gac acc aat gcc gaa gat gga aaa ata tta cat 144
Gly Pro Gly Lys Phe Asp Thr Asn Ala Glu Asp Gly Lys Ile Leu His
        35                  40                  45

gaa atg ggg ggc cac ctt tcg gaa gtt cta tta ctt tca ctt gtt gtt 192
Glu Met Gly Gly His Leu Ser Glu Val Leu Leu Leu Ser Leu Val Val
    50                  55                  60

cta tcc gat ttc gca ccc gaa act gcc tct gcg atg tat ctt gtc cta 240
Leu Ser Asp Phe Ala Pro Glu Thr Ala Ser Ala Met Tyr Leu Val Leu
65                  70                  75                  80

cat ttt tcc atc cca caa cga cac acc gat gtt ctg gac tgc gat aaa 288
His Phe Ser Ile Pro Gln Arg His Thr Asp Val Leu Asp Cys Asp Lys
                85                  90                  95

tct caa cta aat cta acc atg ggc gtc aca acc gcc gat gtt ata ccc 336
Ser Gln Leu Asn Leu Thr Met Gly Val Thr Thr Ala Asp Val Ile Pro
            100                 105                 110

gga tcc gtc tgg aat atg ggc aaa tat gtt tgc ata cga ccc gac tgg 384
Gly Ser Val Trp Asn Met Gly Lys Tyr Val Cys Ile Arg Pro Asp Trp
        115                 120                 125

tgg cct tat gaa acg gct gct gtt ctg gct ttg gaa gaa gtt ggg caa 432
Trp Pro Tyr Glu Thr Ala Ala Val Leu Ala Leu Glu Glu Val Gly Gln
    130                 135                 140

gtt aca cgg atc gtc ttg cgg gca ctc cgc gac ttg aca cgc atc tgg 480
Val Thr Arg Ile Val Leu Arg Ala Leu Arg Asp Leu Thr Arg Ile Trp
145                 150                 155                 160

aac gct gcc aca acc act gca ttt ctt gtc tgc ctt gtt aaa gtt gtc 528
Asn Ala Ala Thr Thr Thr Ala Phe Leu Val Cys Leu Val Lys Val Val
                165                 170                 175

cgc gga caa gtc tta caa ggc gtc ata tgg tta ctg cta ata acg ggc 576
Arg Gly Gln Val Leu Gln Gly Val Ile Trp Leu Leu Leu Ile Thr Gly
            180                 185                 190

gtc caa gga                                                          585
Val Gln Gly
        195

<210> 8
<211> 195
<212> PRT
<213> Bovine viral diarrhea virus

<400> 8
Ser Ser Pro Tyr Cys Asp Val Glu Arg Lys Leu Gly Tyr Ile Trp Phe
1               5                   10                  15

Thr Lys Asn Cys Thr Pro Ala Cys Leu Pro Lys Asn Thr Lys Ile Val
            20                  25                  30

Gly Pro Gly Lys Phe Asp Thr Asn Ala Glu Asp Gly Lys Ile Leu His
        35                  40                  45

Glu Met Gly Gly His Leu Ser Glu Val Leu Leu Leu Ser Leu Val Val
    50                  55                  60

Leu Ser Asp Phe Ala Pro Glu Thr Ala Ser Ala Met Tyr Leu Val Leu
65                  70                  75                  80

His Phe Ser Ile Pro Gln Arg His Thr Asp Val Leu Asp Cys Asp Lys
                85                  90                  95

Ser Gln Leu Asn Leu Thr Met Gly Val Thr Thr Ala Asp Val Ile Pro
            100                 105                 110

Gly Ser Val Trp Asn Met Gly Lys Tyr Val Cys Ile Arg Pro Asp Trp
        115                 120                 125

Trp Pro Tyr Glu Thr Ala Ala Val Leu Ala Leu Glu Glu Val Gly Gln
    130                 135                 140

Val Thr Arg Ile Val Leu Arg Ala Leu Arg Asp Leu Thr Arg Ile Trp
145                 150                 155                 160

Asn Ala Ala Thr Thr Thr Ala Phe Leu Val Cys Leu Val Lys Val Val
                165                 170                 175

Arg Gly Gln Val Leu Gln Gly Val Ile Trp Leu Leu Leu Ile Thr Gly
            180                 185                 190

Val Gln Gly
        195

<210> 9
<211> 1122
<212> DNA
<213> Bovine viral diarrhea virus

<220>
<221> CDS
<222> (1)..(1122)
<223> Protein E2

<400> 9
cgc ctc gat tgc aaa cct gac ttc tca tat gcc att gcc aaa aat gaa     48
Arg Leu Asp Cys Lys Pro Asp Phe Ser Tyr Ala Ile Ala Lys Asn Glu
1               5                   10                  15

aaa att gga cca ctg ggg gct gaa gga ctt act acc act tgg tat gaa     96
Lys Ile Gly Pro Leu Gly Ala Glu Gly Leu Thr Thr Thr Trp Tyr Glu
            20                  25                  30

tac tct gat ggg atg caa ctt tcc gac act atg gtt gaa gct cga tgc     144
Tyr Ser Asp Gly Met Gln Leu Ser Asp Thr Met Val Glu Ala Arg Cys
        35                  40                  45

aaa gat ggg gaa ttt aca ttc atc caa aaa tgc aaa acg gaa acc cga     192
Lys Asp Gly Glu Phe Thr Phe Ile Gln Lys Cys Lys Thr Glu Thr Arg
    50                  55                  60

tat ctg gcc acc ttg cac aca cgg gcc tta ccg aca tct gtc gtt ttt     240
Tyr Leu Ala Thr Leu His Thr Arg Ala Leu Pro Thr Ser Val Val Phe
65                  70                  75                  80

gaa aaa ctt ttt gat gga aat aaa ttg gcg gac atc gtt gaa atg gat     288
Glu Lys Leu Phe Asp Gly Asn Lys Leu Ala Asp Ile Val Glu Met Asp
                85                  90                  95

gac aac ttc gaa ttt gcg atc tgc ccc tgc gat gca aaa ccc gtc gtc     336
Asp Asn Phe Glu Phe Ala Ile Cys Pro Cys Asp Ala Lys Pro Val Val
            100                 105                 110

cgc ggg aaa ttt aac aca aca cta cta aat ggg ccc gcc ttc caa atg     384
Arg Gly Lys Phe Asn Thr Thr Leu Leu Asn Gly Pro Ala Phe Gln Met
        115                 120                 125

gtc tgc ccc att gga tgg act gga tct gtc tcc tgc acc cta gcc aat     432
Val Cys Pro Ile Gly Trp Thr Gly Ser Val Ser Cys Thr Leu Ala Asn
    130                 135                 140

aaa gac acc ctc gat acg gcc gtc gtc cgg aca tat aaa cgc gtt tcc     480
Lys Asp Thr Leu Asp Thr Ala Val Val Arg Thr Tyr Lys Arg Val Ser
145                 150                 155                 160

cca ttc cct aat cgg caa gga tgc gtt act caa aaa ctt ctc ggg gaa     528
Pro Phe Pro Asn Arg Gln Gly Cys Val Thr Gln Lys Leu Leu Gly Glu
                165                 170                 175

gat ctt tat gat tgc atc ttg ggc gga aac tgg act tgc atc gaa ggg     576
Asp Leu Tyr Asp Cys Ile Leu Gly Gly Asn Trp Thr Cys Ile Glu Gly
            180                 185                 190

gaa caa cta cga tac act ggg ggc acc att gaa tcc tgc aag tgg tgc     624
Glu Gln Leu Arg Tyr Thr Gly Gly Thr Ile Glu Ser Cys Lys Trp Cys
        195                 200                 205

ggc tac aaa ttc ttg aaa tcg gaa ggg cta cca cac tat cca att ggc     672
Gly Tyr Lys Phe Leu Lys Ser Glu Gly Leu Pro His Tyr Pro Ile Gly
    210                 215                 220

aaa tgc cgc tta caa aat gaa act ggc tac cgg ctt gtc gac gac acc     720
Lys Cys Arg Leu Gln Asn Glu Thr Gly Tyr Arg Leu Val Asp Asp Thr
225                 230                 235                 240

tct tgc aat gtc ggc ggc gtc gca att gtc cca cat gga ctt gtc aaa     768
Ser Cys Asn Val Gly Gly Val Ala Ile Val Pro His Gly Leu Val Lys
                245                 250                 255

tgc aaa att ggg gat acc gtc gtc caa gtc gtc gca atg gac acg aaa     816
Cys Lys Ile Gly Asp Thr Val Val Gln Val Val Ala Met Asp Thr Lys
            260                 265                 270

ctt gga cct atg cct tgc aaa cca cat gaa ata ata tca tcg gaa gga     864
Leu Gly Pro Met Pro Cys Lys Pro His Glu Ile Ile Ser Ser Glu Gly
        275                 280                 285

ccc gtt gaa aaa acg gca tgc aca ttc aac tat aca cgg acc tta acg     912
Pro Val Glu Lys Thr Ala Cys Thr Phe Asn Tyr Thr Arg Thr Leu Thr
    290                 295                 300

aac aaa tat ttt gaa ccc cgg gac aat tac ttc caa caa tac atg cta     960
Asn Lys Tyr Phe Glu Pro Arg Asp Asn Tyr Phe Gln Gln Tyr Met Leu
305                 310                 315                 320

aaa ggg gac tac caa tat tgg ttt gat ctg gaa gtc tct gac cac cat     1008
Lys Gly Asp Tyr Gln Tyr Trp Phe Asp Leu Glu Val Ser Asp His His
                325                 330                 335

cgg gat tac ttt acg gaa ttc cta ctt gtc att gtt gtc gcc ctc ttg     1056
Arg Asp Tyr Phe Thr Glu Phe Leu Leu Val Ile Val Val Ala Leu Leu
            340                 345                 350

ggc gga cgc tat gtc ctt tgg cta ctt gtc aca tac atg gtc ctc tcc     1104
Gly Gly Arg Tyr Val Leu Trp Leu Leu Val Thr Tyr Met Val Leu Ser
        355                 360                 365

gaa caa aat gcc tcg gct                                             1122
Glu Gln Asn Ala Ser Ala
    370

<210> 10
<211> 374
<212> PRT
<213> Bovine viral diarrhea virus

<400> 10
Arg Leu Asp Cys Lys Pro Asp Phe Ser Tyr Ala Ile Ala Lys Asn Glu
1               5                   10                  15

Lys Ile Gly Pro Leu Gly Ala Glu Gly Leu Thr Thr Thr Trp Tyr Glu
            20                  25                  30

Tyr Ser Asp Gly Met Gln Leu Ser Asp Thr Met Val Glu Ala Arg Cys
        35                  40                  45

Lys Asp Gly Glu Phe Thr Phe Ile Gln Lys Cys Lys Thr Glu Thr Arg
    50                  55                  60

Tyr Leu Ala Thr Leu His Thr Arg Ala Leu Pro Thr Ser Val Val Phe
65                  70                  75                  80

Glu Lys Leu Phe Asp Gly Asn Lys Leu Ala Asp Ile Val Glu Met Asp
                85                  90                  95

Asp Asn Phe Glu Phe Ala Ile Cys Pro Cys Asp Ala Lys Pro Val Val
            100                 105                 110

Arg Gly Lys Phe Asn Thr Thr Leu Leu Asn Gly Pro Ala Phe Gln Met
      115                 120                 125

Val Cys Pro Ile Gly Trp Thr Gly Ser Val Ser Cys Thr Leu Ala Asn
    130                 135                 140

Lys Asp Thr Leu Asp Thr Ala Val Val Arg Thr Tyr Lys Arg Val Ser
145                 150                 155                 160

Pro Phe Pro Asn Arg Gln Gly Cys Val Thr Gln Lys Leu Leu Gly Glu
                165                 170                 175

Asp Leu Tyr Asp Cys Ile Leu Gly Gly Asn Trp Thr Cys Ile Glu Gly
            180                 185                 190

Glu Gln Leu Arg Tyr Thr Gly Gly Thr Ile Glu Ser Cys Lys Trp Cys
      195                 200                 205

Gly Tyr Lys Phe Leu Lys Ser Glu Gly Leu Pro His Tyr Pro Ile Gly
    210                 215                 220

Lys Cys Arg Leu Gln Asn Glu Thr Gly Tyr Arg Leu Val Asp Asp Thr
225                 230                 235                 240

Ser Cys Asn Val Gly Gly Val Ala Ile Val Pro His Gly Leu Val Lys
                245                 250                 255

Cys Lys Ile Gly Asp Thr Val Val Gln Val Val Ala Met Asp Thr Lys
            260                 265                 270

Leu Gly Pro Met Pro Cys Lys Pro His Glu Ile Ile Ser Ser Glu Gly
      275                 280                 285

Pro Val Glu Lys Thr Ala Cys Thr Phe Asn Tyr Thr Arg Thr Leu Thr
    290                 295                 300

Asn Lys Tyr Phe Glu Pro Arg Asp Asn Tyr Phe Gln Gln Tyr Met Leu
305                 310                 315                 320

Lys Gly Asp Tyr Gln Tyr Trp Phe Asp Leu Glu Val Ser Asp His His
                325                 330                 335

Arg Asp Tyr Phe Thr Glu Phe Leu Leu Val Ile Val Val Ala Leu Leu
            340                 345                 350

Gly Gly Arg Tyr Val Leu Trp Leu Leu Val Thr Tyr Met Val Leu Ser
      355                 360                 365

Glu Gln Asn Ala Ser Ala
    370